(19) Europäisches Patentamt
European Patent Office
Office européen des brevets

(11) EP 2 779 845 B1

(12) **EUROPEAN PATENT SPECIFICATION**

(45) Date of publication and mention
of the grant of the patent:
**01.05.2019 Bulletin 2019/18**

(21) Application number: **12787032.7**

(22) Date of filing: **19.11.2012**

(51) Int Cl.:
*A61K 36/28* [(2006.01)]        *A61P 3/04* [(2006.01)]
*A61P 3/06* [(2006.01)]        *A61P 3/10* [(2006.01)]
*A23L 33/105* [(2016.01)]        *A23L 33/00* [(2016.01)]

(86) International application number:
**PCT/EP2012/072984**

(87) International publication number:
**WO 2013/072522 (23.05.2013 Gazette 2013/21)**

(54) **CHICORY EXTRACT AND METHOD OF PREPARING**

CHICORÉEEXTRAKT UND VERFAHREN ZUR HERSTELLUNG

EXTRAIT DE CHICORÉE ET PROCédé de préparation

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.11.2011 EP 11306518**

(43) Date of publication of application:
**24.09.2014 Bulletin 2014/39**

(73) Proprietor: **Naturex S.A.**
**84911 Avignon (FR)**

(72) Inventors:
• **CHAPAL, Nicolas**
**F-34980 Combaillaux (FR)**
• **BEEJMOHUN, Vickram**
**F-34000 Montpellier (FR)**

(74) Representative: **Potter Clarkson**
**The Belgrave Centre**
**Talbot Street**
**Nottingham NG1 5GG (GB)**

(56) References cited:
**US-A1- 2005 003 026    US-A1- 2011 015 140**

• **Joanna Milala ET AL: "Composition and properties of chicory extracts rich in fructans and polyphenols", Polish journal of food and nutrition sciences, 1 January 2009 (2009-01-01), pages 35-43, XP055021223, Retrieved from the Internet: URL:https://encrypted.google.com/#hl=en&output=search&sclient=psy-ab&q=composition+and+properties+of+chicory+extracts+irch+in+fructans+and+polyphenols&oq=composition+an d+properties+of+chicory+extracts+irch+in+fructans+and+polyphenols&aq=f&aqi=&aql=&gs_ sm=3&gs_upl=2674I13575I0I13695I79I62I0I16I 16I0I212I6 [retrieved on 2012-03-07]**
• **Adam Jurgonski ET AL: "Composition of Chicory Root, Peel, Seed and Leaf Ethanol Extracts and Biological Properties of Their Non-Inulin Fractions", Food Technology and Biotechnology, 1 March 2011 (2011-03-01), pages 40-47, XP055021227, Retrieved from the Internet: URL:www.hrcak.srce.hr/file/98058 [retrieved on 2012-03-07]**
• **MULINACCI N ET AL: "Optimization of the chromatographic determination of polyphenols in the aerial parts of Cichorium intybus L", CHROMATOGRAPHIA, VIEWEG UND TEUBNER VERLAG, DE, vol. 54, no. 7-8, 1 January 2001 (2001-01-01), pages 455-461, XP007908705, ISSN: 0009-5893, DOI: 10.1007/BF02491199**

**(Cont. next page)**

- CONFORTI F ET AL: "The protective ability of Mediterranean dietary plants against the oxidative damage: The role of radical oxygen species in inflammation and the polyphenol, flavonoid and sterol contents", FOOD CHEMISTRY, ELSEVIER LTD, NL, vol. 112, no. 3, 1 February 2009 (2009-02-01), pages 587-594, XP023905373, ISSN: 0308-8146, DOI: 10.1016/J.FOODCHEM.2008.06.013 [retrieved on 2008-06-18]
- MARZIA INNOCENTI ET AL: "Evaluation of the Phenolic Content in the Aerial Parts of Different Varieties of Cichorium intybus L.", JOURNAL OF AGRICULTURAL AND FOOD CHEMISTRY, vol. 53, no. 16, 1 August 2005 (2005-08-01), pages 6497-6502, XP055021231, ISSN: 0021-8561, DOI: 10.1021/jf050541d
- I. Massoud Mona ET AL: "Chemical and Technological Studies on Chicory (Cichorium Intybus L) and Its Applications in Some Functional Food", Crop. Processing Res. Dept. Food Technol. Res. Inst. Egypt . 3 Special Foods & Nutrition Dept, Res. Inst. A.R.C.Cairo, 1 January 2009 (2009-01-01), pages 735-756, XP55277488, Retrieved from the Internet: URL:http://education.nu.edu.sa/documents/4 6892/18591832/%D8%A8%D8%AD%D8%AB%20 %D8%A7% D9%84%D8%B4%D9%8A%D9%83%D9%88%D8 %B1%D9%8A% D8%A7.pdf/bd2f4253-b68f-4de1-9bc3-9a3690bc e325 [retrieved on 2016-06-02]

**Description**

The present invention relates to a chicory extract for allowing control of body weight or of body weight gain, reduction or limitation of increase of fat storage, of fatty liver, of liver triglycerides level, of hypertriglyceridemia, of hyperglycemia, of hyperinsulinemia, of insulin resistance, of pre-diabetes and/or of metabolic syndrome.
More particularly, the extract may exhibit an action on risk factor(s) which may have an impact on fatty cirrhosis, cardiovascular disease, diabetes complications and/or diabetes. Among these risk factors, may be cited the overweight, in particular obesity, the increasing of fat storage, of fatty liver, of liver triglycerides level, of hypertriglyceridemia, of hyperglycemia, of hyperinsulinemia, of insulin resistance and/or of different factors of metabolic syndrome.

**Background of the Invention**

The modern society leads more and more to the increase of risk factors of diseases such as fatty cirrhosis, cardiovascular disease, diabetes complications and/or diabetes.
The known compounds or compositions which may be used to treat these diseases and/or to limit one or several of their risk factors, in particular the reduction of body weight, may be insufficiently efficient, too expensive, exhibiting undesired side effects, having insufficient organoleptic qualities, they may change the colour, taste, and/or aspect of the food it is intended to be combined with, difficult to introduce into food, at least with some types of food, insufficiently stable, may present a low solubility, may be insufficiently versatile, may present a sourcing not stable enough, and/or abundant, or they may come from a precursor or precursors which may have other uses.
On the other hand the diets for regulating body weight often have a limited success. Low caloric diets for example may cause a temporary loss of body weight but have not proven their efficiency on the long term for people wanting to lose weight and to maintain a defined weight.
The invention thus aims to solve all or part of the above cited problems. US2005/0003026 describes powder or extracts of plant leaves with anti-obesity effects and anti-obesity food comprising such powder or extracts.
Milala., J. et al, Pol. J. Food. Nutri. Sci., 2009, vol. 59, No.1, pages 35 to 43 describes the composition and properties of chicory extracts rich in fructans and polyphenols.
Jurgonski, A., et al, Food Technol. Biotechnol. 49, (1), pages 40 to 47 describes the composition of chicory root, peel, seed and leaf ethanol extracts and the biological properties of their non-inulin fractions.
Mulinacci, N., et al, Chromatographia, 2001, 54, Pages 455 to 461 describes the optimisation of chromatographic investigations into the polyphenols present in the aerial parts of Cichorium intybus. L.
Conforti, F et al., Food Chemistry, vol. 112, no. 3, 2009, pages 587 to 594 describes the protective activity of Mediterranean plants against oxidative damage.
Innocenti, M., et al J. Agric. Food Chem. 2005, 53, pages 6497 to 6502 describes the evaluation of the phenolic components of the aerial parts of different varieties of Cichorium intybus L.

**General Description of the Invention**

According to an aspect, the invention has for subject matter an extract from a plant of the *Cichorium genus* comprising phenol derivatives, including the three major phenolic acids derivatives identified as chicoric acid, caftaric acid and chlorogenic acid and including flavonoids of Quercetin, and Luteolin according to Claim 1.
The invention may show all or part of the following advantages:

-   very low cost, furthermore it may value a part of a plant which is not used until now, in particular the leaves, but which is easily collected,
-   strong biological effect,
-   food grade quality of the extract, thus leading to the possibility of a use as preventive agent (as it has no or very little toxicity), and/or
-   easiness to obtain an active agent.

Other advantages will be shown in the following description.
The extract may be intended for allowing reduction or control of body weight or limitation of body weight gain, reduction or limitation of increasing of fat storage, of fatty liver, of liver triglycerides level, of hypertriglyceridemia, of hyperglycemia, of hyperinsulinemia, of insulin resistance, pre-diabetes and/or of metabolic syndrome.
The extract may be used in a curative or prophylactic treatment of control of body weight, and/or of weight gain, and/or of increased fat storage, and/or of fatty liver, and/or of high liver triglycerides level, of pre-diabetes and/or of hypertriglyceridemia, and/or for hyperglycemia, and/or of hyperinsulinemia, and/or of insulin resistance, and/or of metabolic syndrome

**Brief Description of the Figures:**

**Figure1** shows HPLC chromatograms of hydro-alcoholic plant extracts at 280 nm
**Figure 2** shows the effect of chicory extracts of the invention on blood glucose in obese pre-diabetic mouse.
**Figure 3** presents the effect of chicory extracts of the invention on blood insulin and insulin resistance in obese pre-diabetic mouse.
**Figure 4** shows the effect of chicory extracts of the invention on body weight and fat storage in obese pre-diabetic mouse.
**Figure 5** presents the effect of chicory extracts of the invention on fatty liver or hepatic steatosis in obese pre-diabetic mouse.
**Figure 6** shows the dose-effect of chicory extracts of the invention on blood glucose in obese pre-diabetic mouse.
**Figure 7** shows the effect of a chicory extract low in chicoric acid, and of a chicory extract of the invention on blood glucose level in obese pre-diabetic mouse.
**Figure 8** shows the effect of purified chicoric acid on blood glucose level in obese pre-diabetic mouse.
**Figure 9** shows the effect of an Echinacea leaf extract, and of a chicory extract of the invention on blood glucose level in obese pre-diabetic mouse.
**Figure 10** shows the effect of an Echinacea leaf extract, and of a chicory extract of the invention on blood insulin and insulin resistance in obese pre-diabetic mouse.

**Detailed Description of the Invention**

The extract of a plant of the *Cichorium genus* of the invention may come from the following species, and mixtures thereof:

- *Cichorium intybus* L.,
- *Cichorium intybus* var. *foliosum,*
- *Cichorium intybus* var. *sativum,*
- *Cichorium intybus* ssp. *intybus* var. *sativum,* and
- *Cichorium endivia* L.

The plant of the *Cichorium genus* is preferably chosen among the plants of the *Cichorium intybus* species, particularly the variety sativum and the *Cichorium endivia* species, and mixtures thereof.
The extract is obtained from the aerial part of the plant, and more particularly from the leaves. Thus the extract may be advantageously a leaf extract.
The expressions "*weight of an extract of a plant of the Cichorium genus*" or "*weight of extract*" in the sense of the invention mean the dry weight of the extract of a plant of the *Cichorium genus* or the dry weight of the extract.
The extract of a plant of the *Cichorium genus* according to the invention comprises phenolic acids and flavonoids. The phenolic acids comprise the three major phenolic acids identified as chicoric acid, caftaric acid and chlorogenic acid. The flavonoids are flavonoids of Quercetin, and Luteolin particularely quercetin 3-*O*-glucuronide, isoquercitrin, and luteoline 7-O-glucuronide.
The amount of chicoric acid in the extract is more than or equal to 30 g/kg of extract, and still more particularly of more or equal to 45 g/kg of extract. In particular embodiments, the amount of chicoric acid in the extract may be up to about 65 to 70 g/kg, depending mainly upon the chicory being used for preparing the extract. In general the amount of chicoric acid in the extract will be ranging from 45 to 55 g/kg.
The amount of caftaric acid in the extract is advantageously of more or equal to 2.5 g/kg of extract, more particularly of more or equal to 4 g/kg of extract, and still more particularly of more or equal to 5 g/kg of extract. In particular embodiments, the amount of caftaric acid in the extract may be up to about 10 g/kg, depending mainly upon the chicory being used for preparing the extract. In general the amount of caftaric acid in the extract will be ranging from 5 to 6 g/kg.
The amount of chlorogenic acid in the extract is advantageously of more or equal to 1 g/kg of extract, more particularly of more or equal to 1.5 g/kg of extract, and still more particularly of more or equal to 2 g/kg of extract. In particular embodiments, the amount of chlorogenic acid in the extract may be up to about 10 g/kg, depending mainly upon the chicory being used for preparing the extract. In general the amount of chlorogenic acid in the extract will be ranging from 5 to 6 g/kg.
The extract may comprise an amount of caftaric acid plus chicoric acid of more or equal to 30 g / kg of extract, more particularly of more or equal to 40 g / kg of extract, and still more particularly of more or equal to 50 g / kg of extract, preferably ranging from 50 to 60 g / kg.

The weight ratio chicoric acid / caftaric acid may range from 2 to 20, in particular from 3 to 15, more particularly from 4 to 14, and still more particularly from 6 to 12, advantageously from 7 to 11.

The weight ratio chicoric acid + caftaric acid / chlorogenic acid may range from 5 to 60, in particular from 6 to 40, more particularly from 7 to 50, and still more particularly from 8 to 20, advantageously from 9 to 19.

The amount of flavonoids in the extract, expressed in terms of the corresponding standard of quercetin, is advantageously of more or equal to 4.5 g/kg of extract, more particularly of more or equal to 5 g/kg of extract, and still more particularly of more or equal to 6.5 g/kg of extract, preferably ranging from 6.5 to 10 g/kg.

The amount of flavonoids may be determined using quercetin as standard, based on its UV spectrum. In other word, the amount of flavonoids is the weight of flavonoids, in particular quercetin, expressed in terms of the corresponding standard of quercetin.

The extract advantageously comprises a total phenolic content ranging from 3 to 10 % by weight total phenol content equivalent to gallic acid.

The total phenolic content in the extract may be determined according to the method using Folin-Ciocalteu reagent and/or also by reversed phase HPLC.

The method of preparing the extract according to the invention from the *Cichorium genus* raw material has to follow a process adapted to result in an active extract comprising the phenolic acids derivatives and the flavonoids derivatives as defined above and in the examples.

Particularly, the method has to take into consideration the stability of the phenolic acids derivatives and the flavonoids derivatives. Purification of the extract has to be performed taking into account the sensitivity of the phenol derivatives to certain reactants such as polyphenols oxidases, since phenolic acids derivatives and flavonoid derivatives are sensitive to said enzymes. The person skilled in the art shall identify such components to be avoided in the extraction and purification process, based on his own skills and knowledges and based on the disclosure of the specific embodiments of the invention.

The extract according to the invention is a hydro-ethanolic extract. The extract may in particular be the extract obtained or obtainable via the method disclosed in this description.

The invention also concerns an extract of a plant of the *Cichorium genus,* such as disclosed in claim 1 and optionally a carrier.

The carrier may particularly be an edible carrier usually used for the preparation of pharmaceutical compositions, nutraceutical compositions and dietary or food supplements. The carrier may comprise one or more flavour agents.

The invention also concerns a method for preparing an extract of a plant of the *Cichorium genus* as defined in claim 1, which extraction process comprises:

a) contacting crushed *Cichorium genus* plant(s) with an extraction solvent,
b) filtering out the solids and collecting the solvent extract,
c) optionally washing the solids by stirring with an extraction solvent,
d) optionally filtering the solids and collecting the solvent extract,
e) combining the solvent extracts and removing non-soluble residues,
f) concentrating the solvents,
g) optionally enriching the phenolic compounds comprising the extract, in particular by precipitation using food grade alcohol,
h) filtering and evaporating the filtrate, and
i) recovering the dry extract, wherein the extraction solvent comprises ethanol/water in a range from 60/40 to 40/60 v/v.

The extraction process of the invention is conducted with the aerial parts of the plants, and more preferably with the leaves.

The plant of the *Cichorium genus* is used dried or fresh and blanched in step a.

Extraction in step a is advantageously performed at a temperature above 25°C, particularly above 35°C, more particularly above or equal to 50°C.

In particular, the extraction in step a is advantageously performed at a temperature above 25°C, more particularly above 35°C, and still more particularly of around 50°C.

Advantageously, the extraction in step a is advantageously performed at a temperature between 25°C and 70°C, preferably between 40°C and 60°C.

The extraction solvent is water/ethanol in a range from 60/40 to 40/60, and even more particularly of around 50/50.

The percentage of alcohol, and in particular ethanol, used for extraction can have an impact on the yield and composition of the biologically active compounds.

The extraction may be done by batch or continuously, or by successive uses of a same extraction solvent in successive batches to obtain saturation of the solvent with the extract. For batch extraction, the person skilled in the art shall define appropriate weight ratio solvent/solid parts of the plant, optimized for an industrial process. The ratio solvent/solid ratio may be ranging from 2 to 20, in particular 5 to 15 and more particularly around 10 times the weight of solids. The extraction may be done one or several time, in particular 2 to 4 times.

Low ratios may be used in continuous or successive batch extraction processes.

The method of the invention may be performed at a temperature ranging from 25 to 70°C, in particular from 40 to 60°C, and more particularly around 50°C.

The extraction may last from 1 to 5 hours, in particular around 2 hours, under stirring, for example mechanical or magnetic stirring.

The remaining solids may be filtered out, in particular through a filter bag.

Several extracts may be combined to form a single extract according to the invention.

The wet solids may be extracted another time by stirring with a solvent, in particular a hydro-alcoholic mixture, with a volume from 1 to 100 times the weight of the dry solids, and a stirring for about 10 to120 minutes further.

The solids are collected and the extracts may be combined.

The different extraction solutions may be combined and left for decantation, filtered through filter paper or centrifugation to remove non-soluble residues.

The clear supernatants obtained may be concentrated to about 5 % to 20 % of their initial volume, for example using a concentrator, and may be then treated with food grade alcohol, in particular ethanol, in a definite proportion, for example a minimum of 2 times the concentrated volume, to remove any precipitate formed. This step may allow removing all or parts of undesired compounds, such as polysaccharides or water soluble proteins.

A powdered extract may be obtained by drying the concentrated extracts, for example using spray drier, oven at 50-80°C, or vacuum drier.

The dry extract is weighed (g) and the extraction yield is calculated by the formula:

$$\text{Dry Yield \%} = (\text{weight of dry extract/weight dry plant}) \times 100$$

$$\text{Fresh yield \%} = (\text{weight of dry extract/weight of fresh raw plant material}) \times 100$$

More particularly, the plant extract may be obtained by the process comprising the following steps:

a) contacting crushed and pressed plant(s) with 2 to 20 times, in particular around 10 times, their weight with ethanol-water comprising at least 40 % by volume of ethanol, and more particularly a mixture ethanol-water having a 1/1 volume ratio, and stirring, for example for 2 hours, in particular at a temperature above 25°C, more particularly above 35°C, and still more particularly of around 50°C,
b) filtering out the solids and collecting the solvent extract,
c) washing the wet solid by stirring with an extraction solvent, in particular with a volume corresponding to 1 to 100 times the weight of the dry solids, for example for about 15 to 120 minutes, particularly for 15 to 30 minutes.
d) filtering the solids and collecting the solvent extract,
e) combining the extraction solutions and decanting or filtering them to remove non-soluble residues,
f) concentrating the solvents, and enriching the extract by precipitating in ethanol
g) concentrating the filtrate under reduced pressure, and
h) drying, for example by staying in an oven at 50-80 °C under vacuum, for example with vacuum spray drying, or the concentrate may be freezed for lyophilisation, and
i) recovering the dry extract.

In general, the yield of extraction is ranging from 1.5 to 3.5 % by weight of extract compared to the total weight of freshly crushed cake. The yield is ranging from 12 to 17% when using dried leaves.

The plant extract can be prepared on a commercial scale by repeating the extraction process that lead to the isolation of the extract of interest.

Thus small-scale extraction procedure can be scaled up, with optionally additional steps of quality control included to ensure reproducible results for the resulting extracts.

Various extraction processes can be employed. Generally, the extract is obtained by contacting the solid plant(s) with the hydro-ethanolic solvent with adequate mixing and for a period of time sufficient to ensure adequate exposure of the solid plant material to the solvent such that biologically active molecules present in the plant material can be taken up by the solvent.

The solvent extraction process may be selected from direct and continuous (counter-current) extraction types at room temperature or at higher temperature with polar and/or non-polar solvent(s). Adequate contact of the solvent with the plant material can be encouraged by shaking the suspension. The liquid fraction is then separated from the solid (insoluble) matter resulting in the generation of two fractions: a liquid fraction, which is the potential extract, and a solid fraction. Separation of the liquid and solid fractions can be achieved by one or more standard processes known to those skilled in art.

The invention also concerns an extract susceptible to be obtained or obtainable by the process of the invention, said extract comprising phenolic acids and flavonoids as defined in the appended claims.

**Examples**

Example 1: preparation of *Cichorium intybus* L extracts

Materials and methods

Dry aerial parts (100g) of *Cichorium intybus* L. are finely chopped (5-8 mm), before extraction.

Extraction process

The plant is extracted with about 1000 ml of a 50 % ethanol/water (V/V) for 2 hours at 50 °C, under mechanical agitation. At the end of the extraction period, the solids are filtered out through a filter bag (PE-100) or other similar straining material.

The wet solids are extracted one more time by stirring with another 3 volumes of ethanol 50% for about 15 to 30 min further.

The solids are again filtered out and the obtained extracts were combined together and 980 ml of the liquid extract is obtained, left for decantation and filtered through filter paper or centrifugation to remove non-soluble residues.

The extraction solution was concentrated under reduced pressure to 5-20 % of its initial volume and then enriched by precipitating in food grade ethanol. The filtrate is again concentrated and set to dryness. The dry extract is recovered.

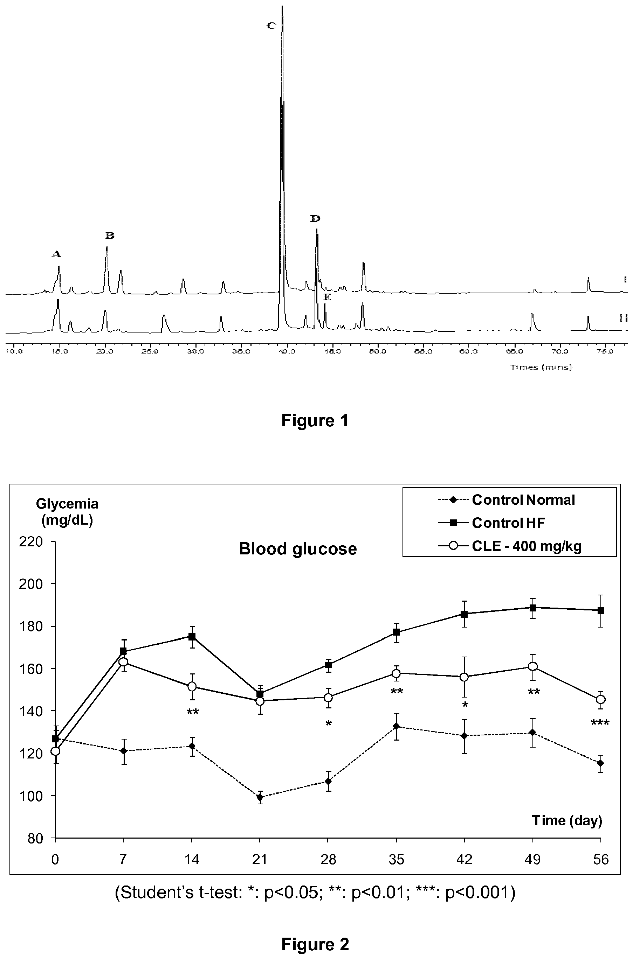

The HPLC chromatograms of the plant extracts at 280 nm of Example 1 is shown in Figure 1 as Chromatogram I, wherein A refers to Caftaric acid, B to Chlorogenic acid, C to Chicoric acid,D to Quercetin 3-O-glucuronide and Isoquercitrin, and E to Luteolin 7-O-glucuronide. Furthermore, the composition of the extract is shown in Table 1 below.

Example 2: preparation of Cichorium intybus ssp.intybus var. sativum extracts

The same procedure than Example 1 was followed except that fresh Cichorium intybus ssp.intybus var. sativum aerial parts was used and blanched before the chopping step.

The HPLC chromatograms of the plant extracts at 280 nm of Example 2 is shown in Figure 1 as Chromatogram II wherein A refers to Caftaric acid, B to Chlorogenic acid C to Chicoric acid, D to Quercetin 3-*O*-glucuronide and Isoquercitrin, and E to Luteolin 7-*O*-glucuronide. Furthermore, the composition of the extract is shown in Table 1 below.

Example 3: composition of the extracts from examples 1 and 2

Analytical Methods

The extracts from examples 1 and 2 comprised between 3 and 7 % by weight total phenol content equivalent to gallic acid, using Folin-Ciocalteu method. Characterization and quantification of the polyphenol content by HPLC:

**- HPLC-DAD Analysis:**

Analytical HPLC (Dionex) is set up as required. In this invention, the mobile phase used was 0.1 % formic acid into 1000 ml high purity water (solvent A) and acetonitrile (solvent B), utilizing the following gradient over a total run of 96 minutes with a flow rate of 0.8 ml/min. The gradients points were for time 0.0 minutes - 95 % A and 5 % B; for time 10 minutes - 90 % A and 10 % B; held isocratic for 10 minutes and from 20 minutes to 40 minutes the gradient varied linearly from 10 % to 20 % B; again held isocratic for 10 minutes; from 50 minutes to 65 minutes the gradient varied linearly from 20 % to 30 % B and to 50 % during the next 10 minutes; from 75 minutes to 76 minutes the gradient varied linearly from 50 % to 100 % B and was held isocratic for 10 minutes. Back to original conditions of 95 % A and 5 % B from 85 minutes to 86 minutes and held isocratic for 10 minutes. Phenolic compounds in the eluent were detected with a UV-diode-array set at 280 nm using a reversed phase C-18 column (250 X 4.6 mm ID X 5μm; ACE). The amounts of phenolic acids in the extracts were determined using calibration curves of: Caftaric acid, Chlorogenic acid, Chicoric acid and the amounts of flavonoids in the extracts were determined using calibration curves of : Quercetin.

- **HPLC-DAD/ESI-MS Analysis:**

HPLC/MS analysis of the extract was performed using an HPLC (Thermo Finnigan surveyor), and interfaced to an LCQ ion trap spectrometer fitted with an electrospray interface (Thermo Finnigan, LCQ Advantage max). The elution program was the same as above, and the experiment was performed in both negative and positive modes. Spectra were scanned over a mass range of m/z 80-2000.

**Table 1**

| Plant extracts | Phenolic acids derivatives | | | Flavonoids derivatives content (D - E) (g Quercetin/kg extract) | Total polyphenol content (g Gallic acid / kg extract) |
|---|---|---|---|---|---|
| | Caftaric acid **(A)** (g/kg extract) | Chlorogenic acid **(B)** (g/kg extract) | Chicoric acid **(C)** (g/kg extract) | | |
| Ex.1 | **5.01 g** | **6,5 g** | **54,52 g** | 6,97 g | **69 g** |
| Ex.2 | **5.97 g** | **2,79 g** | **46,82 g** | **9,91 g** | **54 g** |
| Quantitative composition of extracts from Examples 1 and 2 by HPLC at 280 nm | | | | | |

Example 4: Effect of chicory leaf extract (CLE) on blood glucose (glycemia), blood insulin (insulinemia), insulin resistance, body weight, fat storage and fatty liver Materials and methods

Five-week old C57BL/6NCrl mice (Charles River Laboratories, France) weighing around 20 g were used for the experiment. After 8 days of acclimation, mice were randomly assigned to the different experimental groups (9 animals per group) according to their fasted glycemia.

On Day 0, one group was kept under normal diet (23% of calories from proteins, 66% from carbohydrates, and 11 % from fat) and the others were submitted to high fat diet (17% of calories from proteins, 28% from carbohydrates, and 55% from fat). The diets and water were provided ad libitum. The dosing formulations were administered by oral gavage every morning between 9 h and 10 h from Day 1 to Day 56. The dose volume was 10 ml/kg of body weight. The actual volume administered was calculated and adjusted based on the most recent body weight of each animal. The conditions tested were the followings:

- Control Normal: Mice were fed a normal diet and were administered water once daily.
- Control High Fat: Mice were fed a high fat diet and were administered water once daily.
- CLE 400 mg/kg: Mice were fed a high fat diet and were administered Chicory Leaf extract (CLE) once daily at the dose of 400 mg/kg of body weight.

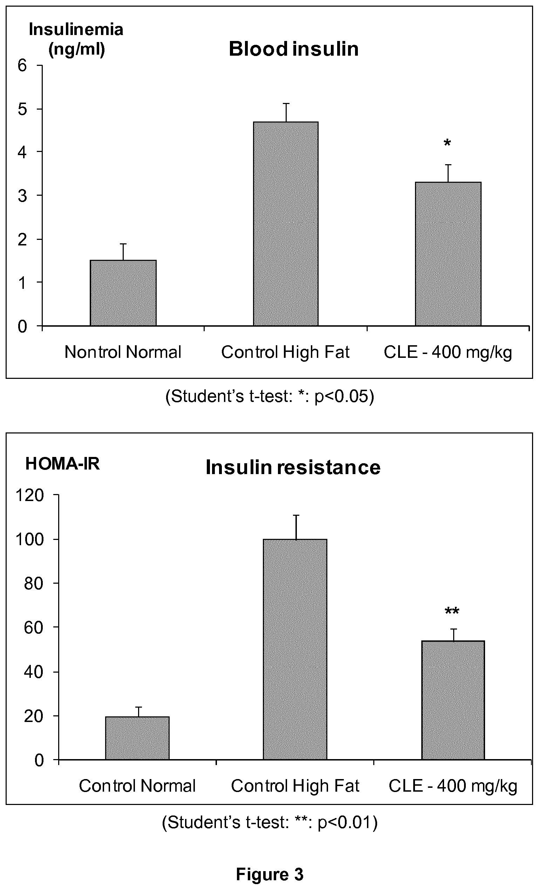

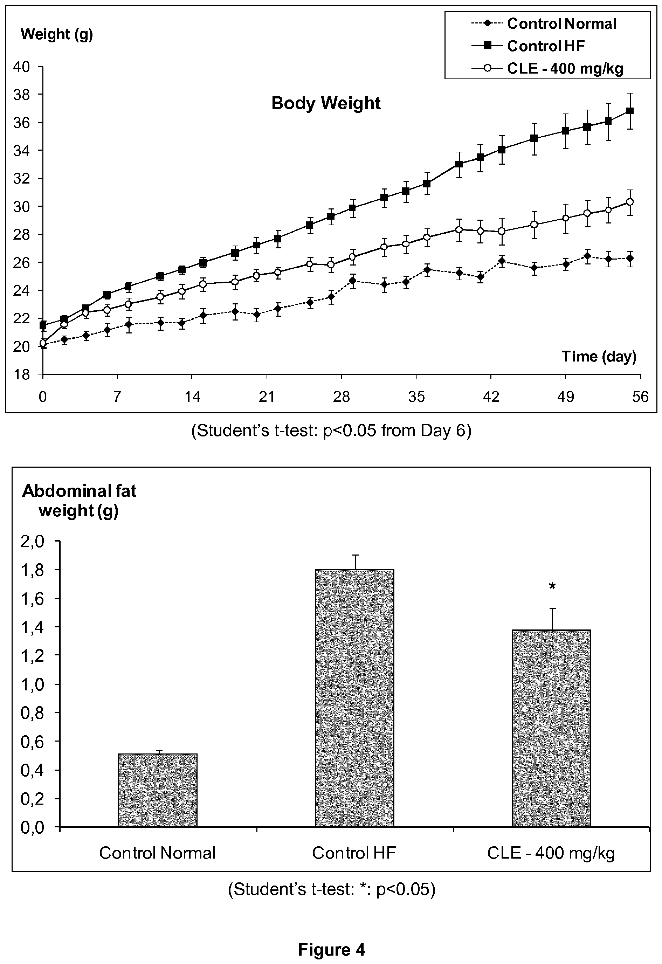

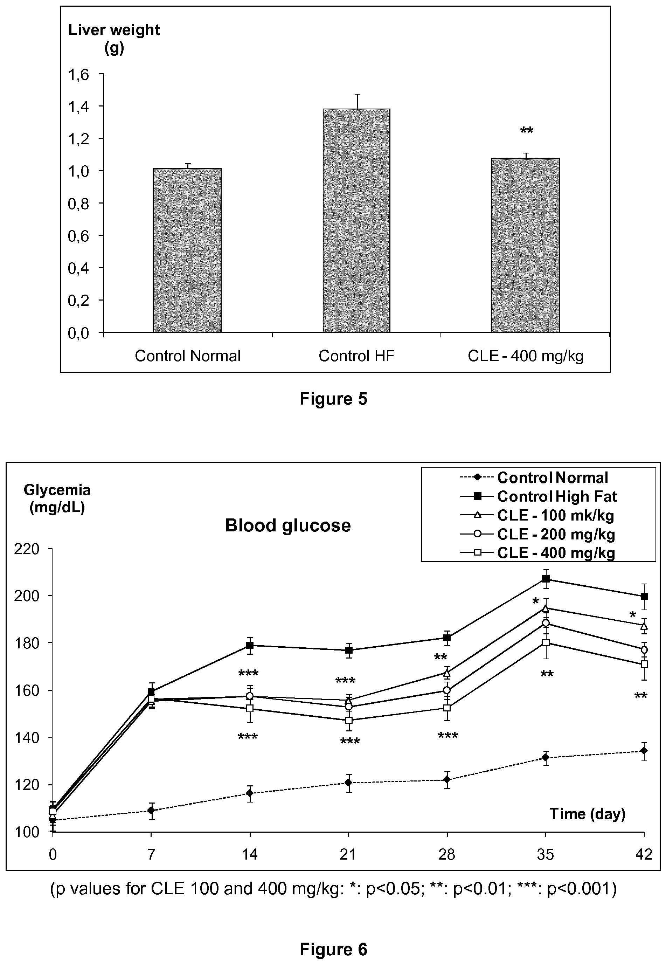

The effect of CLE on glycemia is shown on Figure 2, on insulin and insulin resistance on Figure 3, on body weight and fat storage on Figure 4, and on fatty liver on Figure 5.

Body weights of the mice were recorded at the arrival and then 3 times a week (on Monday, Wednesday, and Friday).

Fasting blood glucose levels were measured the day of randomization (Day 0) and on Day 7, 14, 21, 28, 35, 42, 49, and 56 between 13h00 and 14h00 on animals fasted for 4 hours. Whole blood samples (one drop) were collected via the tail vein for glucose determination using a hand-held glucometer (OneTouch Ultra 2, LifeScan).

At the end of the study, after a 4 hour fast, animals were anaesthetized by an intra peritoneal injection of 0.1 ml of Pentobarbital Sodium. A terminal blood sample was collected via cardiac puncture using heparin as anticoagulant. This terminal blood sampling conducts to the death of the animals. Blood samples were put at 4°C, centrifuged in the 30 min after collection; plasma harvested and kept frozen pending insulin analysis. The epididymal fat pad (abdominal fat) and the liver were harvested to measure their weights.

Fasting plasma insulin levels were measured using an ELISA kit (Mercodia, Sweden). Then, insulin resistance indexes (HOMA-IR) were calculated using the formula: HOMA-IR = fasting insulin (mU/l) x fasting glucose (mmol/l)/22.5.

This protocol was approved by the Regional Ethic Committee (Montpellier, France).

Results

*Blood glucose (Figure 2)*

After one week under diets (Day 7), fasting glycemia of mice submitted to high fat diet was significantly higher (168 ± 5.7 mg/dL) than those of mice submitted to normal diet (121 ± 5.9 mg/dL). Thereafter, this difference stayed rather constant all over the study.

Chicory leaf extract reduced the glycemia of the pre-diabetic mice. At the end of the study (Day 56), glycemia was reduced by 58%. This effect is clearly significant at Day 14, 28, 35, 42, 49, and 56 .

*Blood insulin and insulin resistance (Figure 3)*

Fasting insulinemia was also significantly increased by high fat diet, reaching 4.70 ± 0.43 ng/ml compared to 1.53 ± 0.37 ng/ml for mice under normal diet. In agreement with these increases in fasting glycemia and insulinemia induced by high fat diet, insulin resistance index (HOMA-IR) was multiplied by 5 showing that these animals are strongly insulin resistant.

At the end of the treatment period (Day 56), CLE significantly reduced fasting blood insulin level induced by high fat diet ($p < 0.05$). Insulinemia reached 3.31 ± 0.40 ng/ml for CLE treated mice. Therefore, according to the HOMA-IR insulin resistance index, CLE reduced by 58% insulin resistance induced by high fat diet in pre-diabetic mice ($p < 0.01$).

*Body Weight and abdominal fat weight (Figure 4)*

Mice submitted to high fat diet gained much more weight than those under normal diet. At the end of the study, after 8 weeks of diet, mice under high fat diet gained 2.5 times more weight than the control mice under regular diet. They reached 36.8 ± 1.3 g, whereas mice under normal diet reached 26.3 ± 0.5 g.

This increase of body weight induced by the diet is notably due to the storage of the energy in form of abdominal fat. Indeed, epididymal (abdominal) fat weights of mice under high fat diet were multiplied by 3.5 during the study compared to those of mice under normal diet: 1.80 ± 0.11 and 0.51 ± 0.03 g for high fat and normal diet, respectively. Perivisceral fat is recognized to be associated with or to be a strong risk factor of insulin resistance and metabolic syndrome.

CLE at the dose of 400 mg/kg of body weight reduced body weight gain induced by high fat diet. This effect is significant from Day 4 of treatment and last all along the study ($p < 0.01$ from Day 6). At the end of the study, mice under high fat diet treated with CLE gained 10.1 ± 0.8 g, whereas mice under high fat diet treated with water gained 15.4 ± 1.3 g and mice under normal diet treated with water gained 6.1 ± 0.4 g.

This reduction is at least partly due to a reduction of fat storage in abdominal fat. CLE reduced by 33% fat storage into epididymal fat in mice submitted to high fat diet ($p < 0.05$). Epididymal fat weight of CLE treated mice was 1.38 ± 0.15 g, compared to 1.80 ± 0.11 and 0.51 ± 0.03 g for high fat diet control mice and normal diet control mice, respectively.

*Liver weight (Figure 5)*

High fat diet induced the storage of fat into the liver: what is called fatty liver or hepatic steatosis. The color of the liver became white instead of red for mice under normal diet and its weight was increased by 35%: 1.38 ± 0.10 g for high fat diet versus 1.02 ± 0.03 g for normal diet.

CLE almost normalized fatty liver that is reduced by 83% ($p < 0.01$).

Conclusions

HDF induce a clear phenotype of metabolic syndrome in C57BL/6 mice with a significant increase in body weight gain, abdominal fat storage, fatty liver, fasting glycemia and insulinemia, and insulin resistance.

Chicory leaf extract at the dose of 400 mg/kg of body weight markedly reduced the defects induced by chronic consumption of high fat diet in mice. That comprises:

- Reduction of fasting glycemia
- Reduction of fasting insulinemia
- Reduction of insulin resistance
- Reduction of body weight gain
- Reduction of abdominal fat storage

- Reduction of hepatic steatosis

Therefore, chicory leaf extract is shown to be a good health good ingredient to address one or several metabolic syndrome(s) and related defects.

Example 5: Dose-effect of Chicory Leaf Extracts (CLE) on blood glucose (glycemia) Materials and methods

The protocol was the same than for Example 4.
The conditions tested were the followings:

- Control Normal: Mice were fed a normal diet and were administered water once daily.
- Control High Fat: Mice were fed a high fat diet and were administered water once daily.
- Chicory leaf extract (CLE) 100, 200, or 400 mg/kg: Mice were fed a high fat diet and were administered CLE once daily at the dose of 100, 200, or 400 mg/kg of body weight.

Results

*Blood glucose (Figure 6)*

After one week under diets (Day 7), fasting glycemia of mice submitted to high fat diet was significantly higher (159 ± 3.9 mg/dL) than those of mice submitted to normal diet (109 ± 3.6 mg/dL). Thereafter, this difference stayed rather constant all over the study.
Chicory leaf extract reduced the glycemia of the pre-diabetic mice from Day 14 to Day 42. This effect increased with the dose of CLE and is significant all over the study, for the 3 doses. At the end of the study, the glycemia reached 187 ± 3.2, 177 ± 3.1, 171 ± 6.3 mg/dL for 100, 200, and 400 mg/kg, respectively, compared to 200 ± 5.4 mg/dL for the control.

Conclusions

Chicory leaf extract (CLE) markedly reduced fasting hyperglycemia induced by chronic consumption of high fat diet in mice in a dose-dependent manner. This effect is already significant at the lower dose tested: 100 mg/kg of body weight.

Example 6: Chicory Leaf Extracts (CLE) containing low levels of chicoric acid are less effective to reduce blood glucose

Materials and methods

Low chicoric acid-CLE - Extraction process (not according to the invention) Low chicoric acid-CLE was extracted following the extraction process presented in Example 2 except that the blanching step before chopping was not done. Consequently, the polyphenol oxidases of the plant were not inactivated leading to lower level of polyphenols. Low chicoric acid-CLE contained 1.05% of chicoric acid.

CLE - Extraction process

CLE was extracted following the extraction process presented in Example 2. CLE contained 4.68% of chicoric acid.

Pharmacological protocol

The protocol is comparable to Example 4 but the C57BL/6 mice were submitted to high fat diet for 1 week to induce pre-diabetes before the onset of the treatment. Then, CLE and Low chicoric acid-CLE were given for 3 weeks by oral gavage at the dose of 200 mg/kg of body weight.

Results

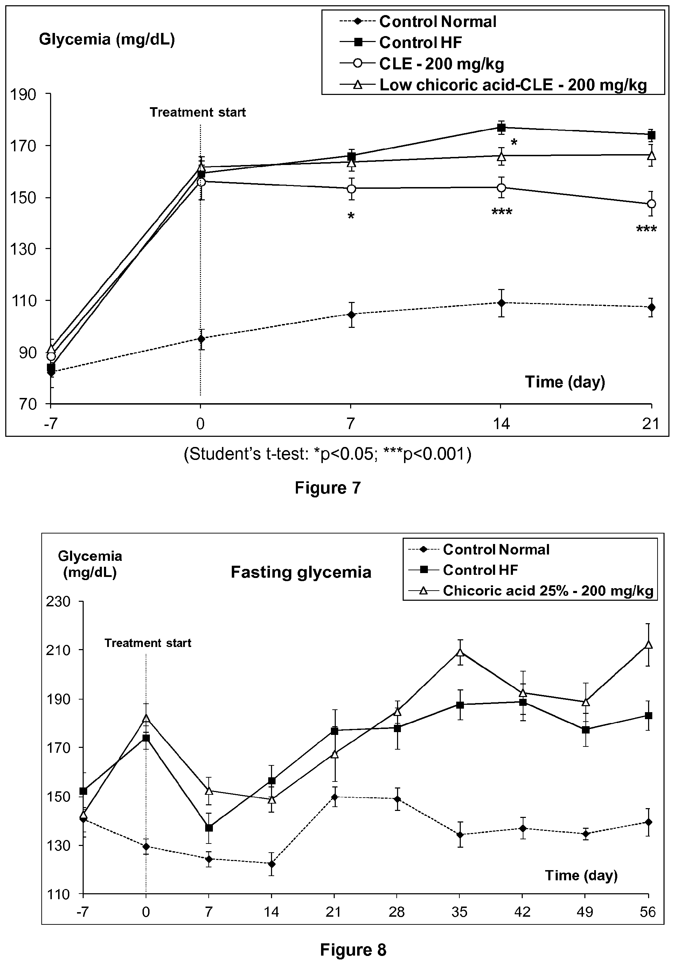

Effect on fasting glycemia (Figure 7)

After one week under diets, fasting glycemia of mice submitted to high fat diet (159.6 ± 4.9 mg/dL) was significantly higher (p<0.001) than those of mice submitted to normal diet (95.2 ± 4 mg/dL). These results confirm the fact that mice under high fat diet were hyperglycemic at the onset of the treatment.

CLE reduced glycemia of the pre-diabetic mice by 31.5% in mean (% of reduction to come back to normal values observed with Control Normal). This effect was clearly significant from the first week of treatment and lasted up to the end of the study (see Figure 7: *p<0.05 at Day 7 and ***p<0.001 at Day 14 and 21).

By contrast, Low chicoric acid-CLE presented a much lower effect. It reduced pre-diabetic mice glycemia by 10.6% in mean. This effect was significant at Day 14 (see Figure 7: *p<0.05) but not at Day 7 and 21.

Conclusions

C57BL/6 mice submitted to high fat diet for 1 week became hyperglycemic. Chicory leaf extract (CLE; containing 4.68% of chicoric acid) at the dose of 200 mg/kg of body weight markedly reduced hyperglycemia and this effect is linked to the presence of sufficient amount of chicoric acid in the extract as Low chicoric acid-CLE (containing 1.05% of chicoric acid) was poorly efficient.

Therefore, chicoric acid is one of the components of CLE that is important for the efficacy of the extract. A sufficient amount of chicoric acid in the extract is necessary to observe an effect on glycemia reduction.

Example 7: Purified chicoric acid (25%) is not effective to reduce blood glucose

Materials and methods

Purified chicoric acid (25%)

Purified chicoric acid was obtained from a commercial source. Briefly, polyphenols extracted from a vegetal source were purified on an adsorbent resin leading to a product rich in polyphenols and containing around 25% of chicoric acid.

Pharmacological protocol

The protocol is comparable to Example 4 but the C57BL/6 mice were submitted to high fat diet for 1 week to induce hyperglycemia before the onset of the treatment. Then, purified chicoric acid at 25% was given for 8 weeks by oral gavage at the dose of 200 mg/kg of body weight.

Results

Effect on fasting glycemia (Figure 8)

At the difference with CLE, purified chicoric acid (25%) presented no effect on fasting glycemia of the pre-diabetic mice even after 8 weeks of treatment (Figure 8).

Conclusions

Therefore, even if chicoric acid is an essential component for CLE efficacy, it is not sufficient. Co-factors, which are suppressed in chicoric acid purification, are also important for the effect of CLE on the reduction of fasting glycemia in pre-diabetic mice.

Example 8: Chicory Leaf Extracts (CLE) is more potent than an Echinacea Leaf Extract (EchLE)

Materials and methods

Echinacea Leaf Extract (EchLE) - Extraction process
EchLE was extracted following the extraction process presented in Example 1. EchLE contained 3.32% of chicoric acid.

Chicory Leaf Extract (CLE) - Extraction process
CLE was extracted following the extraction process presented in Example 2. CLE contained 4.68% of chicoric acid.

Pharmacological protocol

The protocol is identical to Example 6. C57BL/6 mice were submitted to high fat diet for 1 week to induce pre-diabetes before the onset of the treatment. Then, CLE and EchLE were given for 3 weeks by oral gavage at the dose of 200 mg/kg of body weight.

Results

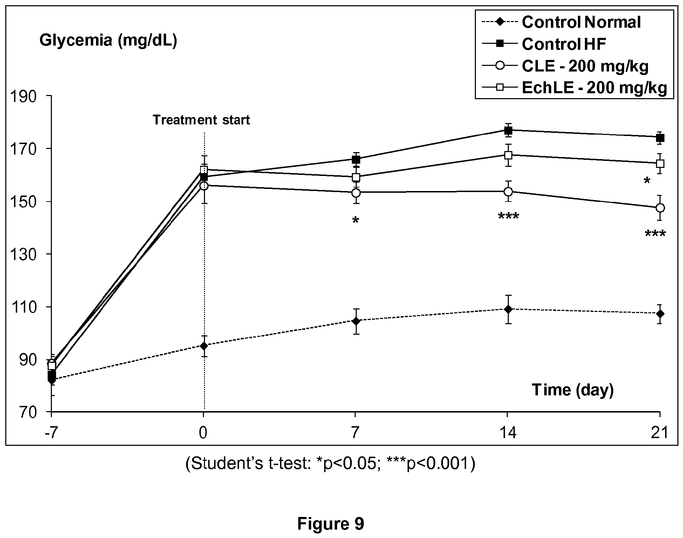

Effect on fasting glycemia (Figure 9)

CLE extract reduced glycemia of the pre-diabetic mice by 31.5% in mean (% of reduction to come back to normal values observed with Control Normal). This effect was clearly significant from the first week of treatment and lasted up to the end of the study (see Figure 9: *$p<0.05$ at Day 7 and ***$p<0.001$ at Day 14 and 21).

EchLE presented a much lower effect. It reduced pre-diabetic mice glycemia by 13.1% in mean. This effect was significant at Day 21 (see Figure 9: *$p<0.05$) but not at Day 7 and 14.

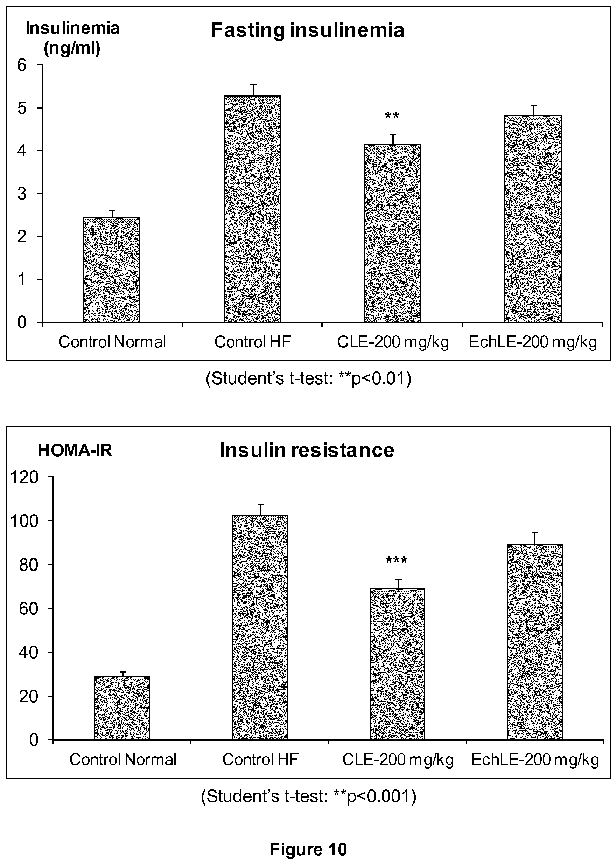

Blood insulin and insulin resistance (Figure 10)

At the end of the treatment period (Day 21), fasting insulinemia was also significantly increased by high fat diet, reaching $5.26 \pm 0.27$ ng/ml compared to $2.43 \pm 0.20$ ng/ml for mice under normal diet. In agreement with these increases in fasting glycemia and insulinemia, insulin resistance index (HOMA-IR) was multiplied by 3, showing that the mice were clearly insulin resistant at the end of the protocol.

CLE significantly reduced fasting blood insulin level induced by high fat diet to $4.16 \pm 0.23$ ng/ml (Figure 10: **$p<0.01$). Therefore, according to the HOMA-IR insulin resistance index, CLE reduced by 45.9% insulin resistance induced by high fat diet in pre-diabetic mice (Figure 10: ***$p<0.001$).

In the same condition, EchLE slightly reduced insulinemia to $4.16 \pm 0.24$ ng/ml but this effect is not statistically significant. Also, insulin resistance was non-significantly decreased by 18.7% (Figure 10).

Conclusions

Chicory leaf extract (CLE; containing 4.68% of chicoric acid), at the dose of 200 mg/kg of body weight, markedly reduced hyperglycemia, hyperinsulinemia, and insulin resistance of pre-diabetic mice. These effects are linked to the presence of sufficient amount of chicoric acid in the extract but also to the chicory origin of the extract as Echinacea leaf extract (EchLE), containing quite high amount of chicoric acid (3.32% of chicoric acid), is much less efficient. Co-factors, present in Cichorium genus but not in Echinacea genus, are important for the effects of CLE.

Taking together, these results show that chicoric acid and co-factors in the extract coming from Cichorium genus are important for the efficacy of CLE.

**Claims**

1. A hydro-ethanolic extract of the aerial parts of a plant of the Cichorium genus comprising the phenolic acids chicoric acid, caftaric acid and chlorogenic acid, and the flavonoids quercetin and luteolin, wherein the amount of chicoric acid is more than or equal to 30 g/kg of the dry weight of the extract, and optionally a carrier, and wherein the extraction solvent is ethanol/water in a range from 60/40 to 40/60 v/v and the aerial parts are dried or are fresh and blanched.

2. The extract according to claim 1, wherein the amount of caftaric acid is of more or equal to 2.5 g / kg of the dry weight of the extract.

3. The extract according to claim 1 or 2, wherein the amount of chlorogenic acid is of more or equal to 1 g / kg of the dry weight of the extract.

4. The extract according to any one of claims 1 to 3, wherein the amount of flavonoids in the extract, expressed in terms of the corresponding standard of quercetin, is of more or equal to 4.5 g/kg of the dry weight of the extract.

5. A method for the preparation of an extract according to any one of claims 1 to 4, said method comprising

   a) contacting crushed aerial parts of a plant of the Cichorium genus with an extraction solvent,
   b) filtering out the solids and collecting the solvent extract,
   c) optionally washing the solids by stirring with an extraction solvent

d) optionally filtering the solids and collecting the solvent extract,
e) combining the solvent extracts and removing non-soluble residues,
f) concentrating the solvents,
g) optionally enriching the phenolic compounds comprising the extract, in particular by precipitation using food grade alcohol,
h) filtering and evaporating the filtrate, and
i) recovering the dry extract;

wherein the method is performed at a temperature ranging from 40 to 60°C and the extraction solvent is ethanol/water in a range from 60/40 to 40/60 v/v and the aerial parts of the plant are dried or are fresh and blanched.

6. The method according to claim 5, wherein the plant of the Cichorium genus is selected from the group consisting of plants of the Cichorium intybus species, plants of the Cichorium endivia species, and mixtures thereof.

7. The method according to any one of claims 5 to 6, wherein the extraction solvent comprises water and ethanol in a volume ratio of water/ethanol of 50/50.

8. An extract according to any one of claims 1 to 4, for use in a treatment selected from the group consisting of:

- a curative or prophylactic treatment of control of body weight, and/or of weight gain,
- a curative or prophylactic treatment of increased fat storage, and/or of fatty liver,
- a curative or prophylactic treatment of pre-diabetes, and
- a curative or prophylactic treatment of metabolic syndrome.

9. An extract of any one of claims 1 to 4, for use in a treatment selected from the group consisting of:

- a curative or prophylactic treatment of high liver triglycerides level,
- a curative or prophylactic treatment of hypertriglyceridemia,
- a curative or prophylactic treatment of hyperglycemia,
- a curative or prophylactic treatment of hyperinsulinemia, and
- a curative or prophylactic treatment of insulin resistance.

## Patentansprüche

1. Hydroethanolisches Extrakt der oberirdischen Teile einer Pflanze der Gattung Cichorium, umfassend die Phenolsäuren Cichoriensäure, Caftarsäure und Chlorogensäure und die Flavonoide Quercetin und Luteol, wobei die Menge an Cichoriensäure wenigstens 30 g/kg des Trockengewichts des Extrakts beträgt, und optional einen Träger, und wobei das Extraktionslösungsmittel Ethanol/Wasser in einem Bereich von 60/40 bis 40/60 Vol./Vol. ist und die oberirdischen Teile getrocknet sind oder frisch und überbrüht sind.

2. Extrakt nach Anspruch 1, wobei die Menge an Caftarsäure wenigstens 2,5 g/kg des Trockengewichts des Extrakts beträgt.

3. Extrakt nach Anspruch 1 oder 2, wobei die Menge an Chlorogensäure wenigstens 1 g/kg des Trockengewichts des Extrakts beträgt.

4. Extrakt nach einem der Ansprüche 1 bis 3, wobei die Menge an Flavonoiden in dem Extrakt, ausgedrückt als der entsprechende Standard von Quercetin, wenigstens 4,5 g/kg des Trockengewichts des Extrakts beträgt.

5. Verfahren zur Herstellung eines Extrakts nach einem der Ansprüche 1 bis 4, wobei das Verfahren Folgendes umfasst:

a) Inberührungbringen zerdrückter oberirdischer Teile einer Pflanze der Gattung Cichorium mit einem Extraktionslösungsmittel,
b) Abfiltrieren der Feststoffe und Sammeln des Lösungsmittelextrakts,
c) optional Waschen der Feststoffe durch Rühren mit einem Extraktionslösungsmittel,
d) optional Filtern der Feststoffe und Sammeln des Lösungsmittelextrakts,
e) Kombinieren der Lösungsmittelextrakte und Entfernen unlöslicher Rückstände,

f) Konzentrieren der Lösungsmittel,
g) optional Anreichern der das Extrakt enthaltenden phenolischen Verbindungen, insbesondere durch Ausfällen unter Verwendung von Alkohol in Lebensmittelreinheit,
h) Filtrieren und Eindampfen des Filtrats und
i) Rückgewinnen des Trockenextrakts;

wobei das Verfahren bei einer Temperatur im Bereich von 40 bis 60 °C durchgeführt wird und das Extraktionslösungsmittel Ethanol/Wasser in einem Bereich von 60/40 bis 40/60 Vol./Vol. ist und die oberirdischen Teile getrocknet sind oder frisch und überbrüht sind.

6. Verfahren nach Anspruch 5, wobei die Pflanze der Gattung Cichorium aus der Gruppe ausgewählt ist, die aus Pflanzen der Cichorium intybus-Art, Pflanzen der Cichorium endivia-Art und Mischungen davon besteht.

7. Verfahren nach einem der Ansprüche 5 bis 6, wobei das Extraktionslösungsmittel Wasser und Ethanol in einem Wasser/Ethanol-Volumenverhältnis von 50/50 umfasst.

8. Extrakt nach einem der Ansprüche 1 bis 4 zur Verwendung bei einer Behandlung, ausgewählt aus der Gruppe bestehend aus:

- einer kurativen oder prophylaktischen Behandlung zur Kontrolle des Körpergewichts und/oder von Gewichtszunahme,
- einer kurativen oder prophylaktischen Behandlung einer erhöhten Fettspeicherung und/oder einer Fettleber,
- einer kurativen oder prophylaktischen Behandlung von Prädiabetes und
- einer kurativen oder prophylaktischen Behandlung von Stoffwechselstörungen.

9. Extrakt nach einem der Ansprüche 1 bis 4 zur Verwendung bei einer Behandlung, ausgewählt aus der Gruppe bestehend aus:

- einer kurativen oder prophylaktischen Behandlung eines hohen Triglyzeridspiegels der Leber,
- einer kurativen oder prophylaktischen Behandlung von Hypertriglyzeridämie,
- einer kurativen oder prophylaktischen Behandlung von Hyperglykämie,
- einer kurativen oder prophylaktischen Behandlung von Hyperinsulinismus und
- einer kurativen oder prophylaktischen Behandlung von Insulinresistenz.

## Revendications

1. Extrait hydroéthanolique des parties aériennes d'une plante du genre *Cichorium* comprenant les acides phénoliques acide chicorique, acide caftarique et acide chlorogénique, et les flavonoïdes quercétine et lutéoline, dans lequel la quantité d'acide chicorique est supérieure ou égale à 30 g/kg du poids sec de l'extrait, et facultativement un support, et dans lequel le solvant d'extraction est l'éthanol/eau dans une gamme de 60/40 à 40/60 v/v et les parties aériennes sont séchées ou sont fraîches et hachées.

2. Extrait selon la revendication 1, dans lequel la quantité d'acide caftarique est supérieure ou égale à 2,5 g/kg du poids sec de l'extrait.

3. Extrait selon la revendication 1 ou 2, dans lequel la quantité d'acide chlorogénique est supérieure ou égale à 1 g/kg du poids sec de l'extrait.

4. Extrait selon l'une quelconque des revendications 1 à 3, dans lequel la quantité de flavonoïdes dans l'extrait, exprimée en termes d'étalon correspondant de quercétine, est supérieure ou égale à 4,5 g/kg du poids sec de l'extrait.

5. Procédé de préparation d'un extrait selon l'une quelconque des revendications 1 à 4, ledit procédé comprenant :

a) la mise en contact des parties aériennes broyées d'une plante du genre *Cichorium* avec un solvant d'extraction,
b) la filtration des solides et la collecte du solvant d'extraction,
c) facultativement le lavage des solides par brassage avec un solvant d'extraction,
d) facultativement la filtration des solides et la collecte de l'extrait de solvant,

e) le mélange des extraits de solvant et l'élimination des résidus non solubles,

f) la concentration des solvants,

g) facultativement l'enrichissement des composés phénoliques comprenant l'extrait, en particulier par précipitation à l'aide d'alcool alimentaire,

h) la filtration et l'évaporation du filtrat, et

i) la récupération de l'extrait sec ;

le procédé étant effectué à une température allant de 40 à 60 °C et le solvant d'extraction étant l'éthanol/eau dans une gamme de 60/40 à 40/60 v/v et les parties aériennes de la plante étant séchées ou étant fraîches et hachées.

6. Procédé selon la revendication 5, dans lequel la plante du genre *Cichorium* est choisie dans le groupe constitué par les plantes de l'espèce *Cichorium intybus,* les plantes de l'espèce *Cichorium endivia* et leurs mélanges.

7. Procédé selon l'une quelconque des revendications 5 à 6, dans lequel le solvant d'extraction comprend de l'eau et de l'éthanol dans un rapport en volume d'eau/éthanol de 50/50.

8. Extrait selon l'une quelconque des revendications 1 à 4, pour une utilisation dans un traitement choisi dans le groupe constitué par :

   - un traitement curatif ou prophylactique de contrôle du poids corporel et/ou de la prise de poids,
   - un traitement curatif ou prophylactique du stockage accru des graisses et/ou de la stéatose hépatique,
   - un traitement curatif ou prophylactique du prédiabète, et
   - un traitement curatif ou prophylactique du syndrome métabolique.

9. Extrait selon l'une quelconque des revendications 1 à 4, pour une utilisation dans un traitement choisi dans le groupe constitué par :

   - un traitement curatif ou prophylactique du taux élevé de triglycérides dans le foie,
   - un traitement curatif ou prophylactique de l'hypertriglycéridémie,
   - un traitement curatif ou prophylactique de l'hyperglycémie,
   - un traitement curatif ou prophylactique de l'hyperinsulinémie, et
   - un traitement curatif ou prophylactique de l'insulinorésistance.

**Figure 1**

(Student's t-test: *: p<0.05; **: p<0.01; ***: p<0.001)

**Figure 2**

(Student's t-test: *: p<0.05)

(Student's t-test: **: p<0.01)

**Figure 3**

(Student's t-test: p<0.05 from Day 6)

(Student's t-test: *: p<0.05)

**Figure 4**

**Figure 5**

(p values for CLE 100 and 400 mg/kg: *: p<0.05; **: p<0.01; ***: p<0.001)

**Figure 6**

(Student's t-test: *p<0.05; ***p<0.001)

**Figure 7**

**Figure 8**

**Figure 9**

(Student's t-test: **p<0.01)

(Student's t-test: **p<0.001)

Figure 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050003026 A

**Non-patent literature cited in the description**

- **MILALA., J. et al.** *Pol. J. Food. Nutri. Sci.,* 2009, vol. 59 (1), 35-43
- **JURGONSKI, A. et al.** *Food Technol. Biotechnol.,* vol. 49 (1), 40-47
- **MULINACCI, N. et al.** *Chromatographia,* 2001, vol. 54, 455-461
- **CONFORTI, F et al.** *Food Chemistry,* 2009, vol. 112 (3), 587-594
- **INNOCENTI, M. et al.** *J. Agric. Food Chem.,* 2005, vol. 53, 6497-6502